# EUROPÄISCHE PATENTSCHRIFT

(11) **EP 1 076 537 B1**
(45) Veröffentlichungstag und Bekanntmachung des Hinweises auf die Patenterteilung: **11.10.2006**
(21) Anmeldenummer: 99923501.3
(22) Anmeldetag: 29.04.1999
(51) Int. Cl.: A61F 2/46

(54) **INSTRUMENT ZUR HANDHABUNG VON KOMPONENTEN VON GELENKPROTHESEN**
INSTRUMENT FOR MANIPULATING COMPONENTS OF JOINT PROSTHESES
INSTRUMENT POUR MANIPULER DES COMPOSANTS DE PROTHESES ARTICULAIRES

(30) Priorität: 30.04.1998 DE 19819193; 11.05.1998 DE 19820721
(43) Veröffentlichungstag der Anmeldung: 21.02.2001
(73) Patentinhaber: CeramTec AG Innovative Ceramic Engineering, 73207 Plochingen (DE)
(72) Erfinder: BÄDORF, Dirk, D-50226 Frechen (DE); KÄLBERER, Hartmut, D-73269 Hochdorf (DE); PFAFF, Hans-Georg, D-73760 Ostfildern (DE); RACK, Robert, D-73207 Plochingen (DE)
(74) Vertreter: Uppena, Franz
(86) Internationale Anmeldenummer: PCT/EP1999/002933
(87) Internationale Veröffentlichungsnummer: WO 1999/056677

(56) Entgegenhaltungen:
- EP-A- 0 373 078
- EP-A- 0 888 759
- WO-A-94/21199
- FR-A- 2 701 206
- US-A- 5 169 399

## Beschreibung

Die Erfindung betrifft ein Instrument zur Handhabung und zum Einsetzen einer Einsatzschale in eine Außenschale einer zweiteiligen Gelenkpfannen-Prothese entsprechend dem Oberbegriff des ersten Anspruchs, wie es z.B. aus der US-A*-*5 169 399 bekannt ist.

Gelenk-Prothesen, bei denen ein Gelenkpartner als Pfanne und der andere Gelenkpartner als Kugelkopf ausgebildet ist, der in der Pfanne drehbar gelagert ist, sind insbesondere als Schulter- und Hüftgelenk-Prothesen bekannt. Diese Prothesen sind in der Regel modular aufgebaut. Das heißt, daß beispielsweise die Gelenkpfanne zweiteilig ist und aus einer Außenschale besteht, die in den Knochen eingesetzt wird und aus einer Einsatzschale, die in die Außenschale eingesetzt wird und die die Gelenkfläche, die Gleitfläche für den anderen Gelenkpartner, aufweist. Das Einsetzen der Einsatzschale in die Außenschale erfolgt in der Regel mittels Preßsitz. Dazu ist es notwendig, daß die Einsatzschale sehr genau zur Außenschale ausgerichtet werden muß, um beim Einsetzen ein Verkanten zu vermeiden. Beim verkanteten Einsetzen können, außer einer ungenauen Zuordnung der Gelenkfläche zum Kugelkopf, Beschädigungen an der Einsatzschale auftreten. Insbesondere Einsatzschalen aus Keramik können ausbrechen oder sogar zerbrechen.

Werden die Einsatzschalen durch den Chirurgen mit der Hand eingesetzt, ist die Handhabung in dem Umfeld der Operationswunde aufgrund der durch Körperflüssigkeiten benetzten Handschuhe des Chirurgen und der schlechten Übersichtlichkeit schwierig. Aus diesem Grund ist es bekannt, Instrumente vorzusehen, mit der einzusetzende Einsatzschalen besser zu handhaben sein sollen. Aus dem deutschen Gebrauchsmuster DE 297 02 093 U1 ist ein Handhabungsinstrument von Gelenkkomponenten mit Gelenkflächen bekannt, das ein Griffteil und daran ein endseitig angeordnetes Saugelement aufweist, welches ein in sich geschlossenes, an die Gelenkfläche der Gelenkkomponente abdichtend anlegbares und mit dieser Gelenkfläche dadurch eine abgeschlossene Saugkammer ausbildendes Dichtelement sowie eine den Druck der Saugkammer absenkende Einrichtung umfaßt. Dieses Handhabungsinstrument wird mit dem Dichtelement auf die Gelenkfläche der einzusetzenden Einsatzschale aufgesetzt, der Druck in der Saugkammer wird erniedrigt und dadurch die Einsatzschale angesaugt. Nach dem Einsetzen der Einsatzschale wird der Druck wieder erhöht und das Handhabungsinstrument löst sich selbsttätig von der eingesetzten Einsatzschale.

Das bekannte Handhabungsinstrument bietet aber keine Sicherheit, daß die Einsatzschale in der erforderlichen Genauigkeit auf die Außenschale aufgesetzt und dadurch ein Verkanten vermieden werden kann. Außerdem sind die bekannten Handhabungsgeräte lang (bis zu 40 cm). Sie sind dadurch unhandlich und lassen somit das erforderliche Feingefühl beim Einsetzvorgang vermissen.

Der vorliegenden Erfindung liegt die Aufgabe zugrunde, ein Instrument zur Handhabung und zum Einsetzen einer Einsatzschale in eine Außenschale einer zweiteiligen Gelenkpfannen-Prothesen vorzustellen, mit dem ein leichtes Positionieren und ein verkantungsfreies Einsetzen möglich ist.

Die Lösung der Aufgabe erfolgt mit Hilfe der kennzeichnenden Merkmale des ersten Anspruchs. Vorteilhafte Ausgestaltungen der Erfindung werden in den Unteransprüchen beansprucht.

Das erfindungsgemäße Instrument zur Handhabung und zum Einsetzen einer Einsatzschale in eine Außenschale einer zweiteiligen Gelenkpfannen-Prothese besteht aus einem Griff mit einem daran anschließenden Haltewerkzeug. Durch den Griff läßt sich das Haltewerkzeug fur die Einsatzschale leicht handhaben. Das Haltewerkzeug weist mindestens zwei Greifklauen auf. Diese erstrecken sich im wesentlichen radial zur Längsachse des Griffs. Die Krallen der Greifklauen liegen auf der stirnseitigen Umfangsflache der einzusetzenden Einsatzschale auf.

Durch die Anordnung der Greifklauen und ihrer Krallen wird die Einsatzschale genau zentrisch zur Längsachse des Griffs und senkrecht zu dieser Achse ausgerichtet gehalten. Dadurch wird für den Chirurgen eine genaue Ausrichtung der Einsatzschale beim Einsetzen möglich. Die kurze Bauart des Instruments bietet außerdem dem Chirurgen das erforderliche Feingefühl beim Einsetzvorgang.

Die Krallen der Greifklauen umgreifen die sich verjüngende Außenfläche der Einsatzschale in jeweils gleicher Länge so weit, daß einerseits ein sicheres Halten aber auch andererseits ein leichtes Ausstoßen aus den Krallen möglich ist. Das Ausstoßen der Einsatzschale aus den Greifklauen des Haltewerkzeugs erfolgt mittels eines Stößels, der verschiebbar in einer Bohrung gelagert ist, die konzentrisch zur Längsachse des Griffs verläuft. Beim Verschieben des Stößels in Richtung auf die Einsatzschale wird diese aus den Krallen der Greifklauen herausgedrückt und kann in die Außenschale der Gelenkpfannen-Prothese eingedrückt werden.

In einer Ausgestaltung der Erfindung weisen die Krallen der Greifklauen senkrecht zur Längsachse verlaufende Stirnflächen auf. Diese Stirnflächen dienen zum Aufsetzen auf die Stirnfläche der Außenschale. Da die Krallen an den Greifklauen alle gleich lang sind, ist beim Aufsetzen aller Klauen auf die Stirnfläche der Außenschale die Einsatzschale für ihren Einsatz genau positioniert. Diese Ausgestaltung der Greifklauen ist ein vorteilhaftes Hilfsmittel für den Chirurgen, mit dem er in dem schwierigen Umfeld der Operationswunde die Einsatzschale sicher und genau für den Einsatz positionieren kann.

Damit bei der Positionierung des Instruments mit der Einsatzschale auf der Außenschale keine Beeinträchtigung der Lage der Einsatzschale erfolgt, ist die Länge der Krallen mindestens so bemessen, daß bei ihrem Aufsetzen auf die Stirnfläche der Außenschale die von ihnen gehaltene Einsatzschale noch keinen Klemmkontakt mit der Außenschale hat. Damit ist ein Korrektur der Positionierung jeder Zeit möglich. Diese ist abgeschlossen, wenn alle Klauen mit ihren Stirnflächen auf der Stirnfläche der Außenschale aufliegen.

In weiterer Ausgestaltung der Erfindung sind die Klauen aus einem elastischen Werkstoff, damit die gehaltene Einsatzschale leicht aus dem Haltewerkzeug herausdrückbar ist. Ein weiterer Vorteil besteht darin, daß Einsatzschalen, die sich in ihren Durchmessern nur geringfügig unterscheiden, von ein und dem selben Haltewerkzeug gehalten werden können.

In einer anderen Ausgestaltung der Erfindung sind die Krallen der Greifklauen aus einem elastischen Werkstoff gefertigt. Insbesondere bei Instrumenten, die nur einmal benutzt werden, können die Krallen so ausgebildet sein, daß sie sich beim Herausdrücken der Einsatzschale aus den Greifklauen sogar bleibend verformen.

In weiterer vorteilhafter Ausgestaltung der Erfindung weist der Griff eine Griffmulde auf. Diese Griffmulde ermöglichst beispielsweise die Klemmung des Griffs zwischen zwei parallelen Fingern, beispielsweise dem Mittel- und dem Ringfinger oder dem Zeige- und dem Mittelfinger. Die Griffmulde kann beispielsweise U-förmig sein und um den gesamten Griff laufen, so daß eine optimale Lagerung des Instruments in der Hand des Chirurgen in jeder möglichen Ausrichtung möglich ist. Das Ausdrücken der Einsatzschale kann dann beispielsweise mittels des Daumens oder des Handballens erfolgen.

Damit der Stößel beim Ausstoßen der Einsatzschale aus den Greifklauen keine punktuelle Belastung auf die Einsatzschale ausübt, ist es vorteilhaft, wenn sein Ende mindestens eine Platte aufweist, mit der er sich ringförmig auf die Einsatzschale abstützt. Eine solche Platte hat den Vorteil, daß der Stößel für Einsatzschalen mit unterschiedlichen Gelenkflächendurchmessern einsetzbar ist.

In einer anderen Ausgestaltung der Erfindung weist der Stößel an seinem Ende einen der Gelenkfläche der Einsatzschale angepaßten Stempelkopf auf. Der Stempelkopf hat dann die Form einer Kugelkalotte, deren Radius dem Radius der Gelenkfläche entspricht. Ein solcher Stößel ist zwar nur für einen Durchmesser einer Einsatzschale einsetzbar, weist aber den Vorteil auf, daß er die beim Einsetzen aufzubringende Kraft großflächig und gleichmäßig auf die Einsatzschale verteilt.

Das Einpressen der Einsatzschale in die Außenschale erfolgt in der Regel durch den Chirurgen manuell. Zur endgültigen Fixierung kann allerdings noch ein nachträglicher Schlag mit einem Schlagwerkzeug erforderlich sein. In einem solchen Fall bietet auch das erfindungsgemäße Instrument eine Möglichkeit, einen solchen Fixierungsschlag gezielt auf die Einsatzschale aufzubringen. Durch die genaue Ausrichtung des Instruments zur Außenschale und damit der Einsatzschale zur Außenschale werden die durch den Schlag aufgebrachten Kräfte gleichmäßig und ihrem Zweck entsprechend genau in die Einsatzschale eingeleitet.

Anhand eines Ausführungsbeispiels wird die Erfindung näher erläutert.

Es zeigen:
- Figur 1: das erfindungsgemäße Instrument mit einer einzusetzenden Einsatzschale im Haltewerkzeug,
- Figur 2: einen Schnitt längs der Achse durch den Griff des Instruments, wie in der Figur eingezeichnet, und
- Figur 3: ein Schnittbild entsprechend Figur 2 mit einer weiteren Ausgestaltung des Stößels.

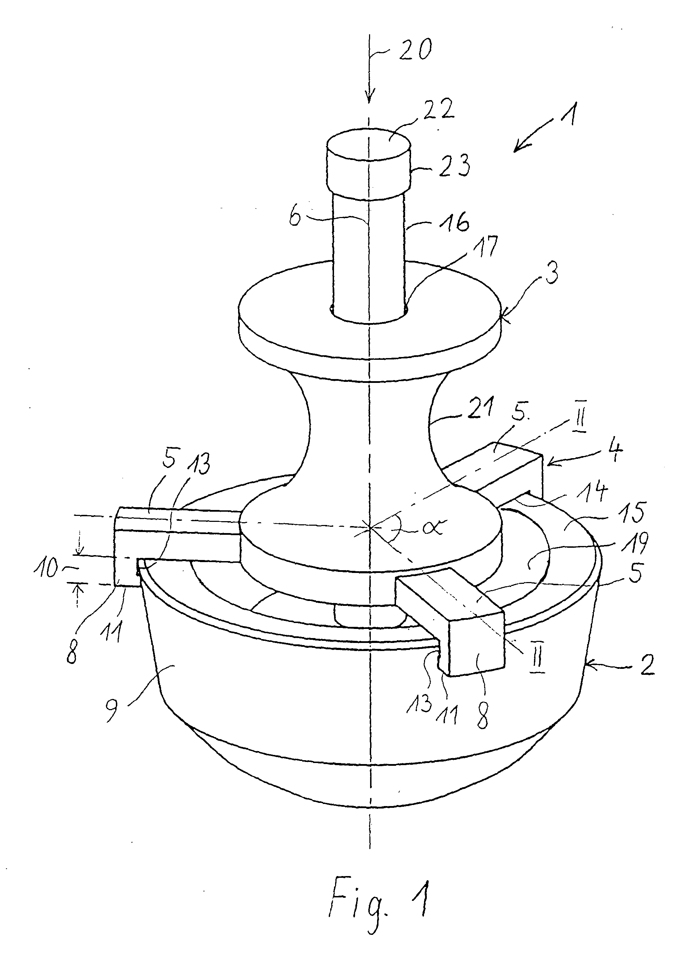

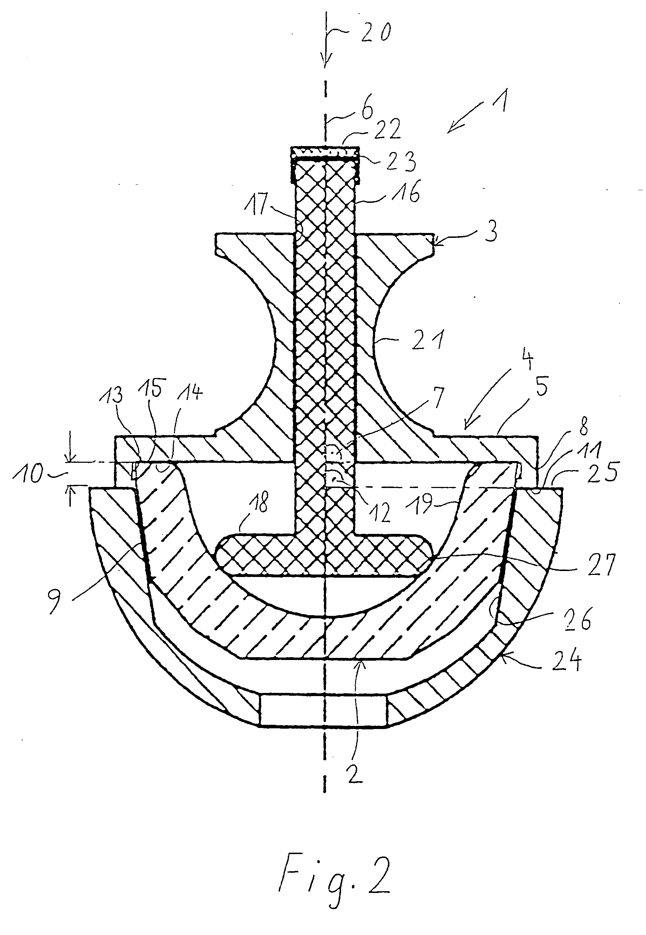

Die Darstellung in den Figuren erfolgt in vergrößertem Maßstab. In Figur 1 ist mit 1 das erfindungsgemäße Instrument zur Handhabung und zum Einsetzen der von ihm gehaltenen Einsatzschale 2 bezeichnet. Das Instrument 1 besteht aus einem-Griff 3 und einem Haltewerkzeug 4. Das Haltewerkzeug 4 weist im vorliegenden Ausführungsbeispiel drei Greifklauen 5 auf, die sich im wesentlichen radial von der Längsachse 6 des Griffs 3 aus erstrecken. Im Schnittbild der Figur 2 ist das anhand des rechten Winkels 7 ersichtlich. Die Greifklauen sind im vorliegenden Ausführungsbeispiel in gleichen Winkelabständen → angeordnet, was aber nicht zwingend erforderlich ist.

Die Greifklauen 5 weisen Kralle 8 auf, die die sich verjüngende Außenfläche 9 der Einsatzschale 2 übergreifen. Um der Konizität der Außenfläche 9 der Einsatzschale 2 Rechnung zu tragen, weisen die Krallen 8 jeweils eine Hinterschneidung 13 auf. Die Krallen 8 weisen alle die gleiche Länge 10 auf. Damit eine genaue Positionierung und Ausrichtung der Einsatzschale 2 zur Außenschale möglich ist, stehen die Anlageflächen 14 der Greifklauen 5, die auf der Stirnseite 15 der Einsatzschale 2 liegen, senkrecht zur Längsachse 6 des Griffs 3. Ihre Verlängerung schneidet die Achse 6 im rechten Winkel 7, wie in Figur 2 dargestellt. Das ermöglicht eine genaue Anlage des Instruments an die Stirnfläche der Außenschale, wie aus der Figur 2 ersichtlich ist.

Im vorliegenden Ausführungsbeispiel sind die Greifklauen 5 elastisch, so daß bei einem Druck auf die Einsatzschale 2 in Richtung aus dem Instrument 1 heraus die Krallen 8 nach außen gebogen werden und die Einsatzschale 2 freigeben.

Das Ausdrücken der Einsatzschale 2 aus dem Haltewerkzeug 4 erfolgt mittels eines Stößels 16. Der Stößel 16 ist in einer Bohrung 17 gelagert, die konzentrisch zur Längsachse 6 im Griff 3 verläuft. An seinem, der Einsatzschale 2 zugewandten Ende trägt der Stößel 16 im vorliegenden Ausführungsbeispiel eine Platte 18. Diese liegt mit ihrem äußeren Umfang 27 ringförmig an der Gelenkfläche 19 der Einsatzschale 2 an. Die Anlagefläche liegt konzentrisch zur Längsachse 6 des Griffs 3, wie aus der Figur 2 ersichtlich. Dadurch wird eine gleichmäßige Einleitung der Druckkraft in die Einsatzschale 2 gewährleistet. Wird auf den Stößel 16 in Pfeilrichtung 20 eine Kraft ausgeübt, so geben die Greifklauen 5 aufgrund ihrer Elastizität die Einsatzschale 2 frei.

Der Griff 3 besitzt eine U-förmige Griffmulde 21, die zentrisch um den Griff umläuft. Sie ist so gestaltet, daß der Griff insbesondere zwischen zwei parallel verlaufende Finger gehalten werden kann. Dann ist es möglich, mittels des Handballens oder mit dem Daumen auf die Stirnfläche 22 des Stößels 16 zu drücken und die Einsatzschale 2 aus dem Haltewerkzeug 4 herauszudrücken. Eine Kappe 23 auf der Stirnfläche 22 des Stößels 16 besitzt einen solchen Durchmesser, daß der Stößel nicht durch die Bohrung 17 hindurchgleiten kann, wenn die Einsatzschale 2 in die Außenschale gedrückt wird. Außerdem schützt diese Kappe 23 den Schaft 22 bei eventuellen Schlägen mit einem Schlagwerkzeug und verhindert so ein Abplatzen oder Absplittern der Stirnseitenkanten des Stößels 16.

Figur 2 zeigt einen Längsschnitt durch das Instrument 1 und der von ihr gehaltenen Einsatzschale 2 in der Positionierposition auf einer Außenschale 24. Der Schnittverlauf ist in Figur 1 mit II-II eingezeichnet. Er verläuft durch zwei benachbarte Greifklauen 5.

In Figur 2 ist das Instrument 1 mit der von ihm gehaltenen Einsatzschale 2 mit den Stirnflächen 11 der Krallen 8 auf die Stirnfläche 25 der Außenschale 24 aufgesetzt. Die Einsatzschale 2 ist in der Außenschale 24 positioniert. Beim Aufsetzen aller Stirnflächen 11 der Krallen 8 auf die Stirnfläche 25 der Außenschale 24 ist eine genaue Zentrierung und Positionierung der Einsatzschale 2 gewährleistet.

Wie aus dem Schnittbild ersichtlich, ubergreifen die Krallen 8 die Außenfläche 9 der Einsatzschale 2 in ihrem Randbereich in gleicher Länge 10. Diese Länge stimmt mit der Länge der Krallen 8 überein. Die Länge 10 der Krallen 8 ist so gewählt, daß dann, wenn ihre Stirnflächen 11 auf der Stirnfläche 25 der Außenschale 24 vollständig aufliegen, noch kein Klemmkontakt zwischen der Außenfläche 9 der Einsatzschale 2 und der Innenfläche 26 der Außenschale 24 besteht.

Erst durch Betätigung des Stößels 16 durch eine Krafteinwirkung in Pfeilrichtung 20 werden die Greifklauen 5 aufgrund ihrer Elastizität die Einsatzschale 2 freigeben. Im vorliegenden Ausführungsbeispiel trägt der Stößel 16 an seinem der Einsatzschale 2 zugewandten Ende eine Platte 18, die auf ihrem äußeren Umfang 27 ringförmig auf der Gelenkfläche 19 der Einsatzschale 2 liegt. Durch eine Krafteinwirkung in Pfeilrichtung 20 auf den Stößel 16 wird die Einsatzschale 2 nach Freigabe durch das Haltewerkzeug 4 in die Außenschale 24 gepreßt. Aufgrund der Konizitäten der Außenfläche 9 der Einsatzschale 2 und der Innenfläche 26 der Außenschale 24 erfolgt ein Preßsitz.

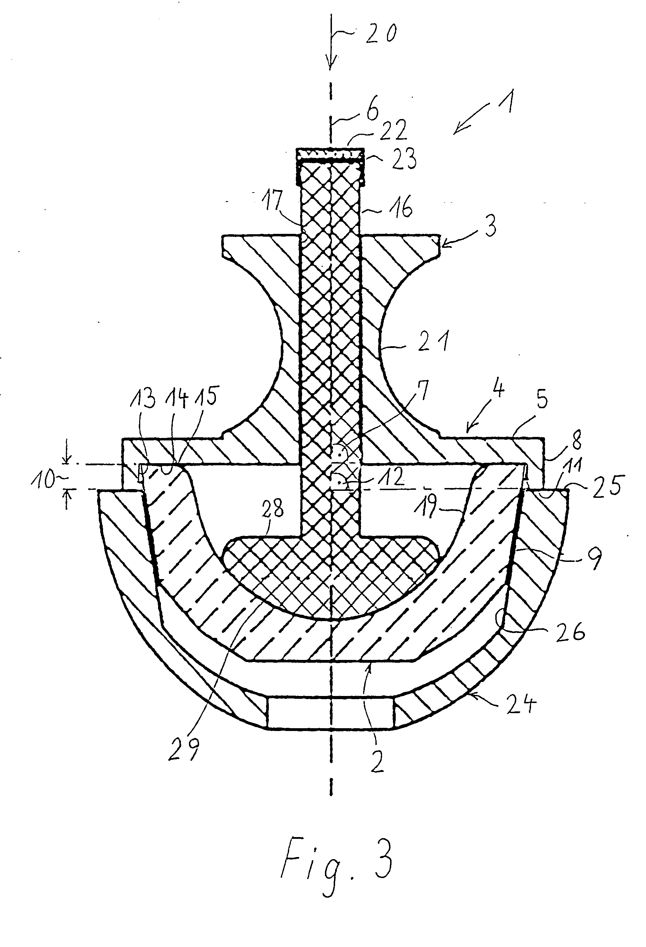

In Figur 3, das ein der Figur 2 entsprechendes Schnittbild wiedergibt, trägt der Stößel 16 an seinem, der Einsatzschale 2 zugewandten Ende einen der Gelenkfläche 19 angepaßten Stempelkopf 28. Gleichartige Merkmale sind in diesem Ausführungsbeispiel mit denselben Bezugszeichen bezeichnet. Der Stempelkopf 28 hat die Form einer Kugelkalotte und seine Oberfläche 29 entspricht genau der der Gelenkfläche 19 der Einsatzschale 2. Bei dieser Ausführungsform ist eine großflächige und gleichmäßige Einleitung der Kraft in die Einsatzschale 2 möglich.

Das Instrument 1 zur Handhabung einer Einsatzschale 2 kann aus einem sterilisierbaren Material hergestellt werden, beispielsweise aus einem hitze- und chemikalienresistenten Kunststoff oder aus einem Metall mit gleichen Eigenschaften, so daß eine Wiederverwendung möglich ist. Das Instrument kann aber auch als ein Einmalwerkzeug angesehen werden, das nach seiner Verwendung entsorgt wird. In diesem Fall ist es möglich, eine Einsatzschale zusammen mit einem Instrument, das die Einsatzschale bereits mit seinem Haltewerkzeug hält, steril verpackt dem Chirurgen zur Verfügung zu stellen. Damit entfällt das manuelle Einsetzen der Einsatzschale in das Haltewerkzeug des Instruments während der Operation, was die Arbeitsbedingungen des Chirurgen erleichtert und Beschädigungen der empfindlichen Gelenkfläche der Einsatzschale ausschließt.

## Patentansprüche

1. Instrument zur Handhabung und zum Einsetzen einer Einsatzschale in eine Außenschale einer zweiteiligen Gelenkpfannen-Prothese mit einem Haltewerkzeug zum lösbaren Halten der einzusetzenden Einsatzschale, wobei das Instrument (1) aus einem Griff (3) und einem sich daran anschließenden Haltewerkzeug (4) besteht, und das Haltewerkzeug (4) mindestens zwei Greifklauen (5) aufweist, die sich im wesentlichen radial (7) von der Längsachse (6) des Griffs (3) erstrecken, **dadurch gekennzeichnet, daß** die Greifklauen (5) dazu geeignet sind, auf der Stirnfläche (15) der Einsatzschale (2) aufzuliegen, daß die Greifklauen (5) Krallen (8) aufweisen, die dazu geeignet sind, die sich verjüngende Außenfläche (9) der Einsatzschale (2) in jeweils gleicher Länge (10) so weit zu übergreifen, daß eine Freigabe der Einsatzschale (2) bei einer auf ihr ausgeübten Druckkraft erfolgt, daß durch den Griff (3) konzentrisch zu seiner Längsachse (6) eine Bohrung (17) verläuft, in der ein aus dem Griff (3) herausragender Stößel (16) verschiebbar gelagert ist, und daß beim Verschieben des Stößels (16) in Richtung (20) auf die Einsatzschale (2) diese aus dem Haltewerkzeug (4) herausdrückbar ist.

2. Instrument nach Anspruch 1, **dadurch gekennzeichnet, daß** die Krallen (8) der Greifklauen (5) des Haltewerkzeugs (4) senkrecht (12) zur Längsachse (6) des Griffs (3) verlaufende Stirnflächen (11) zum Aufsetzen auf die Stirnfläche (25) der Außenschale (24) aufweisen.

3. Instrument nach Anspruch 1 oder 2 in Kombination mit einer Einsatzschale und einer Außenschale, **dadurch gekennzeichnet, daß** die Länge (10) der Krallen (8) mindestens so bemessen ist, daß beim Aufsetzen der Einrichtung (1) auf die Stirnfläche (25) der Außenschale (24) die konische Außenfläche (9) der Einsatzschale (2) noch keinen Klemmkontakt mit der Innenfläche (26) der Außenschale (24) hat.

4. Instrument nach einem der Ansprüche 1 bis 3, **dadurch gekennzeichnet, daß** die Greifklauen (5) des Haltewerkzeugs (4) aus einem elastischen Werkstoff bestehen.

5. Instrument nach einem der Ansprüche 1 bis 3, **dadurch gekennzeichnet, daß** die Krallen (8) der Greifklauen (5) des Haltewerkzeugs (4) aus einem elastischen Werkstoff bestehen.

6. Instrument nach einem der Ansprüche 1 bis 5, **dadurch gekennzeichnet, daß** der Griff (3) des Instruments (1) eine Griffmulde (21) aufweist.

7. Instrument nach Anspruch 6, **dadurch gekennzeichnet, daß** die Griffmulde (21) U-förmig und umlaufend ist.

8. Instrument nach einem der Ansprüche 1 bis 7, **dadurch gekennzeichnet, daß** der Stößel (16) an seinem der Einsatzschale (2) zugewandten Ende eine Platte (18) aufweist, die mit ihrem äußeren Umfang (27) konzentrisch an der Gelenkfläche (19) der Einsatzschale (2) anlegbar ist.

9. Instrument nach einem der Ansprüche 1 bis 8, **dadurch gekennzeichnet, daß** der Stößel (16) an seinem der Einsatzschale (2) zugewandtem Ende einen der Gelenkfläche (19) angepaßten Stempelkopf (28) trägt.

## Claims

1. An instrument for handling an insert shell and for inserting it into an outer shell of a two-part joint socket prosthesis, the instrument having a holding tool for detachably holding the insert shell that is to be inserted, wherein the instrument (1) comprises a handle (3) and a holding tool (4) connected thereto, and the holding tool (4) has at least two gripping dogs (5) which extend substantially radially (7) from the longitudinal axis (6) of the handle (3), **characterised in that** the gripping dogs (5) are adapted to rest on the end face (15) of the insert shell (2), **in that** the gripping dogs (5) have claws (8) which are adapted to grip over the tapering outer surface (9) of the insert shell (2) by the same length (10) in each case to the extent that release of the insert shell (2) takes place when a pressing force is exerted on it, **in that** a bore (17) extends through the handle (3), concentrically with respect to its longitudinal axis (6), in which bore (17) is mounted, in a sliding manner, a plunger (16) projecting from the handle (3), and **in that** when the plunger (16) is slid in the direction (20) of the insert shell (2), the latter can be pushed out of the holding tool (4).

2. An instrument according to claim 1, **characterised in that** the claws (8) of the gripping dogs (5) of the holding tool (4) have end faces (11) for placing on the end face (25) of the outer shell (24) that extend at right angles (12) to the longitudinal axis (6) of the handle (3).

3. An instrument according to claim 1 or 2 in combination with an insert shell and an outer shell, **characterised in that** the length (10) of the claws (8) is at least dimensioned in such a way that when the device (1) is placed onto the end face (25) of the outer shell (24), the conical outer surface (9) of the insert shell (2) still has no clamping contact with the inner surface (26) of the outer shell (24).

4. An instrument according to one of claims 1 to 3, **characterised in that** the gripping dogs (5) of the holding tool (4) consist of an elastic material.

5. An instrument according to one of claims 1 to 3, **characterised in that** the claws (8) of the gripping dogs (5) of the holding tool (4) consist of an elastic material.

6. An instrument according to one of claims 1 to 5, **characterised in that** the handle (3) of the instrument (1) has a recessed grip (21).

7. An instrument according to claim 6, **characterised in that** the recessed grip (21) is U-shaped and circumferential.

8. An instrument according to one of claims 1 to 7, **characterised in that** on the end that faces the insert shell (2), the plunger (16) has a plate (18), the outer circumference (27) of which can be placed concentrically on the joint surface (19) of the insert shell (2).

9. An instrument according to one of claims 1 to 8, **characterised in that** on the end that faces the insert shell (2), the plunger (16) has a stamping head (28) which is matched to the joint surface (19).

## Revendications

1. Instrument pour manipuler et pour introduire une coquille d'insertion dans une coquille extérieure d'une prothèse à coussinet à rotule en deux parties, en utilisant un outil de maintien pour maintenir de manière amovible la coquille d'insertion à mettre en place, l'instrument (1) étant composé d'une poignée (3) et d'un outil de maintien (4) se raccordant à celle-ci et qui comprend au moins deux pinces de saisie partant essentiellement radialement de l'axe longitudinal (6) de la poignée (3), **caractérisé en ce que**
- les pinces de saisie présentent des collets (8) aptes chacun à saisir la surface externe (9) allant en se rétrécissant, de la coquille d'insertion (2), sur une même longueur (10) faisant que la libération de la coquille d'insertion (2) s'effectue en exerçant sur elle une force de poussée,
- la poignée (3) présente concentriquement à son axe longitudinal (6) un alésage (17) dans lequel peut coulisser un poussoir (16) faisant saillie sur la poignée (3), le coulissement de ce poussoir (16) dans la direction (20) de la coquille d'insertion (2) permettant de faire sortir celle-ci de l'outil de maintien (4).

2. Instrument selon la revendication 1, **caractérisé en ce que** les collets (8) des pinces de saisie (5) de l'outil de maintien (4) présentent perpendiculairement (12) à l'axe longitudinal (6) de la poignée (3) des portées frontales (11) à appliquer sur la face frontale (25) de la coquille externe (24).

3. Instrument selon la revendication 1 ou 2, en combinaison avec une coquille d'insertion et une coquille externe, **caractérisé en ce que** la longueur (10) des collets (8) est au moins dimensionnée de manière que lors de l'application de l'instrument (1) sur la face frontale (25) de la coquille externe (24), la surface conique (9) de la coquille d'insertion (2) n'a pas encore un contact de pincement avec la surface interne (26) de la coquille externe (24).

4. Instrument selon une des revendications 1 à 3, **caractérisé en ce que** les pinces de saisie (5) de l'outil de maintien (4) sont en un matériau élastique.

5. Instrument selon une des revendications 1 à 3, **caractérisé en ce que** les collets (8), les pinces de saisie (5) de l'outil de maintien (4) sont en un matériau élastique.

6. Instrument selon une des revendications 1 à 5, **caractérisé en ce que** la poignée (3) de l'instrument (1) présente une gorge concave (21).

7. Instrument selon la revendication 6, **caractérisé en ce que** la gorge concave (21) est périphérique avec une forme en U.

8. Instrument selon une des revendications 1 à 7, **caractérisé en ce que** le poussoir (16), à son extrémité du côté de la coquille d'insertion (2), présente une plaque (18) qui peut s'appliquer concentriquement par sa périphérie externe (27) sur la portée d'articulation (19) de la coquille d'insertion (2).

9. Instrument selon une des revendications 1 à 8, **caractérisé en ce que** le poussoir (16) porte, à son extrémité située vers la coquille d'insertion (2), une tête de poussoir (28) adaptée à la portée d'articulation (19).
